# EUROPEAN PATENT SPECIFICATION

(11) **EP 4 099 898 B1**
(45) Date of publication and mention of the grant of the patent: **28.01.2026**
(21) Application number: 21750131.1
(22) Date of filing: 05.02.2021
(51) Int. Cl.: A61B 5/0215, A61B 5/00

(54) **INTRACARDIAC PRESSURE SENSOR WITH CLIP STRUCTURE**
INTRAKARDIALER DRUCKSENSOR MIT CLIP-STRUKTUR
CAPTEUR DE PRESSION INTRACARDIAQUE AVEC STRUCTURE DE PINCE

(30) Priority: 05.02.2020 US 202062970275 P
(43) Date of publication of application: 14.12.2022
(73) Proprietor: Shifamed Holdings, LLC, Campbell, CA 95008 (US)
(72) Inventor: FOX, William Jason, Campbell, California 95008 (US); SCHRECK, Stefan, Campbell, California 95008 (US); FAHEY, Brian, Campbell, California 95008 (US); ANDRIOLA, Peter, Campbell, California 95008 (US); ROBERTSON, Scott, Campbell, California 95008 (US); ALEXANDER, Miles, Campbell, California 95008 (US); PANTAGES, Anthony, Campbell, California 95008 (US); SAGE, Shahn, Campbell, California 95008 (US)
(74) Representative: Forresters IP LLP
(86) International application number: PCT/US2021/016932
(87) International publication number: WO 2021/159001

(56) References cited:
- US-A1- 2002 072 656
- US-A1- 2002 120 200
- US-A1- 2007 021 664
- US-A1- 2015 223 707
- US-A1- 2019 000 327
- US-A1- 2019 167 197
- US-B1- 10 471 251
- US-B1- 7 450 999

## Description

### TECHNICAL FIELD

The present technology generally relates to implantable medical devices and, in particular, to implantable intracardiac sensors and associated systems and methods for monitoring physiological parameters of a patient's heart.

### BACKGROUND

Heart failure is a medical condition associated with the inability of the heart to effectively pump blood to the body. Heart failure affects millions of people worldwide, and may arise from multiple root causes, but is generally associated with myocardial stiffening, myocardial shape remodeling, and/or abnormal cardiovascular dynamics. Chronic heart failure is a progressive disease that worsens considerably over time. Initially, the body's autonomic nervous system adapts to heart failure by altering the sympathetic and parasympathetic balance. While these adaptations are helpful in the short-term, over a longer period of time they serve to make the disease worse.

Heart failure is a medical term that includes both heart failure with reduced ejection fraction (HFrEF) and heart failure with preserved ejection fraction (HFpEF). The prognosis with both HFpEF and HFrEF is poor; one-year mortality is 26% and 22%, respectively, according to one epidemiology study. In spite of the high prevalence of HFpEF, there remain limited options for HFpEF patients. Pharmacological therapies have been shown to impact mortality in HFrEF patients, but there are no similarly-effective evidence-based pharmacotherapies for treating HFpEF patients. Current practice is to manage and support patients while their health continues to decline.

A common symptom among heart failure patients is elevated left atrial pressure. In the past, clinicians have treated patients with elevated left atrial pressure by creating a shunt between the left and right atria using a blade or balloon septostomy. The shunt decompresses the left atrium (LA) by relieving pressure to the right atrium (RA) and systemic veins. Over time, however, the shunt typically will close or reduce in size. More recently, percutaneous interatrial shunt devices have been developed which have been shown to effectively reduce left atrial pressure. However, these percutaneous devices often have an annular passage with a fixed diameter which fails to account for a patient's changing physiology and condition. For this reason, existing percutaneous shunt devices may have a diminishing clinical effect after a period of time. Many existing percutaneous shunt devices typically are also only available in a single size that may work well for one patient but not another. Also, sometimes the amount of shunting created during the initial procedure is later determined to be less than optimal months later. An example of a prior art document is US2002/120200, which teaches a remote sensor assembly (RSA) with a pressure transducer in one heart chamber and a pressure transmission catheter that extends through a septal wall and includes an opening. Accordingly, there is a need for improved devices, systems, and methods for treating heart failure patients, particularly those with elevated left atrial pressure.

### BRIEF DESCRIPTION OF THE DRAWINGS

FIG. 1 is a schematic illustration of an interatrial device implanted in a heart and configured in accordance with an embodiment of the present technology.
FIGS. 2A-2D illustrate an intracardiac pressure monitoring device configured in accordance with an embodiment of the present technology.
FIGS. 3A-3D illustrate the intracardiac pressure monitoring device of FIGS. 2A-2D being implanted in a patient's heart in accordance with an embodiment of the present technology.
FIGS. 4A and 4B illustrate an interatrial shunting system including the intracardiac pressure monitoring device of FIGS. 2A-2D and configured in accordance with an embodiment of the present technology.
FIG. 5 illustrates an intracardiac pressure monitoring device configured in accordance with another embodiment of the present technology.
FIGS. 6A-6C illustrate a pressure transmission element configured in accordance with an embodiment of the present technology.

### DETAILED DESCRIPTION

The present technology is generally directed to devices for monitoring pressure within a patient's heart. An intracardiac pressure monitoring device configured in accordance with an embodiment of the present technology can include, for example, a clip structure configured to be implanted in the heart (e.g., in the septal wall). The clip structure can include a first clip segment configured to engage a first side of the septal wall (e.g., the left atrial side) and a second clip segment configured to engage a second, opposite side of the septal wall (e.g., the right atrial side). The device can further include one or more pressure sensors for measuring pressure of one or more heart chambers (e.g., the LA and/or RA). For example, the device can include a first pressure sensor operably coupled to a first pressure transmission element carried by the first clip segment and/or a second pressure sensor operably coupled to a second pressure transmission element operably coupled to the second clip segment. In some embodiments, the device is used for dual chamber pressure monitoring, e.g., the first pressure sensor can be configured to measure left atrial pressure and the second pressure sensor can be configured to measure right atrial pressure. In some embodiments, the pressure sensor(s) are mechanically independent from the clip structure but are operably coupled to the clip structure and/or a component mechanically-coupled to the clip structure (e.g., sensors communicate with a processor attached to a clip structure via a wireless signal).

In some embodiments, the intracardiac pressure monitoring devices described herein are implemented as part of an interatrial shunting system. The system can include a shunting element implantable into a patient at or adjacent a septal wall. The shunting element can fluidly connect the LA and the RA of the patient to facilitate blood flow therebetween. The system can further include an intracardiac pressure monitoring device including a clip structure for securing the device to the septal wall. In some embodiments, the system can adjust the shunting element (e.g., the size and/or shape of the shunt lumen) based on pressure measurements generated by the intracardiac pressure monitoring device. The invention is defined by the appended claims.

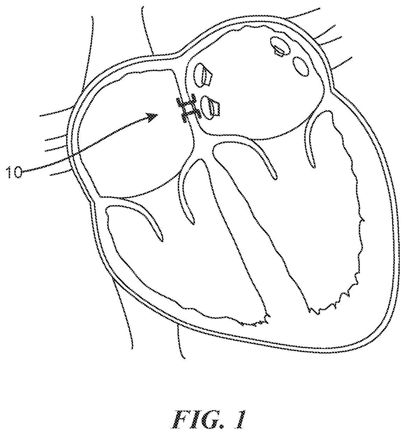

The terminology used in the description presented below is intended to be interpreted in its broadest reasonable manner, even though it is being used in conjunction with a detailed description of certain specific embodiments of the present technology. Certain terms may even be emphasized below; however, any terminology intended to be interpreted in any restricted manner will be overtly and specifically defined as such in this Detailed Description section. Additionally, the present technology can include other embodiments that are within the scope of the examples but are not described in detail with respect to FIGS. 1-6C.

Reference throughout this specification to "one embodiment" or "an embodiment" means that a particular feature, structure, or characteristic described in connection with the embodiment is included in at least one embodiment of the present technology. Thus, the appearances of the phrases "in one embodiment" or "in an embodiment" in various places throughout this specification are not necessarily all referring to the same embodiment. Furthermore, the particular features or characteristics may be combined in any suitable manner in one or more embodiments.

Reference throughout this specification to relative terms such as, for example, "generally," "approximately," and "about" are used herein to mean the stated value plus or minus 10%.

As used herein, the terms "interatrial device," "interatrial shunt device," "IAD," "IASD," "interatrial shunt," and "shunt" are used interchangeably to refer to a device that, in at least one configuration, includes a shunting element that provides a blood flow between a first region (e.g., a LA of a heart) and a second region (e.g., a RA or coronary sinus of the heart) of a patient. Although described in terms of a shunt between the atria, namely the left and right atria, one will appreciate that the technology may be applied equally to devices positioned between other chambers and passages of the heart, or between other parts of the cardiovascular system or other system. For example, any of the shunts described herein, including those referred to as "interatrial," may be nevertheless used and/or modified to shunt between the LA and the coronary sinus, or between the right pulmonary vein and the superior vena cava. Moreover, while the disclosure herein primarily describes shunting blood from the LA to the RA, the present technology can be readily adapted to shunt blood from the RA to the LA to treat certain conditions, such as pulmonary hypertension. For example, mirror images of embodiments, or in some cases identical embodiments, used to shunt blood from the LA to the RA can be used to shunt blood from the RA to the LA in certain patients.

As used herein, the term "clip structure" refers to a component configured to be secured to a site within the patient's heart (e.g., to the septal wall). For example, the disclosed clip structures can apply a compressive force against the septal wall to anchor a pressure monitoring device thereto. The clip structures can also be configured as support structures or support members to which a number of additional components of the disclosed pressure monitoring devices may be operably coupled/attached for operation within the patient. Although certain embodiments of the clip structures described herein are discussed with respect to "preventing" movement of intracardiac pressure monitoring devices relative to a portion of the patient's heart, one of skill in the art will appreciate that such clip structures may still allow for movements that are expected to have little or no detrimental effect on the operation of the pressure monitoring devices (e.g., movements that do not cause the clip structure and/or associated components to become dislodged from the septal wall).

The headings provided herein are for convenience only and do not interpret the scope or meaning of the claimed present technology.

### A. Interatrial Shunts for Treatment of Heart Failure

Heart failure can be classified into one of at least two categories based upon the ejection fraction a patient experiences: (1) HFpEF, historically referred to as diastolic heart failure or (2) HFrEF, historically referred to as systolic heart failure. One definition of HFrEF is a left ventricular ejection fraction lower than 35%-40%. Though related, the underlying pathophysiology and the treatment regimens for each heart failure classification may vary considerably. For example, while there are established pharmaceutical therapies that can help treat the symptoms of HFrEF, and at times slow or reverse the progression of the disease, there are limited available pharmaceutical therapies for HFpEF with only questionable efficacy.

In heart failure patients, abnormal function in the left ventricle (LV) leads to pressure build-up in the LA. This leads directly to higher pressures in the pulmonary venous system, which feeds the LA. Elevated pulmonary venous pressures push fluid out of capillaries and into the lungs. This fluid build-up leads to pulmonary congestion and many of the symptoms of heart failure, including shortness of breath and signs of exertion with even mild physical activity. Risk factors for HF include renal dysfunction, hypertension, hyperlipidemia, diabetes, smoking, obesity, old age, and obstructive sleep apnea. HF patients can have increased stiffness of the LV which causes a decrease in left ventricular relaxation during diastole resulting in increased pressure and inadequate filling of the ventricle. HF patients may also have an increased risk for atrial fibrillation and pulmonary hypertension, and typically have other comorbidities that can complicate treatment options.

Interatrial shunts have recently been proposed as a way to reduce elevated left atrial pressure, and this emerging class of cardiovascular therapeutic interventions has been demonstrated to have significant clinical promise. FIG.1 shows the conventional placement of a shunt in the septal wall between the LA and RA. Most conventional interatrial shunts (e.g., shunt 10) involve creating a hole or inserting a structure with a lumen into the atrial septal wall, thereby creating a fluid communication pathway between the LA and the RA. As such, elevated left atrial pressure may be partially relieved by unloading the LA into the RA. In early clinical trials, this approach has been shown to improve symptoms of heart failure.

One challenge with many conventional interatrial shunts is determining the most appropriate size and shape of the shunt lumen. A lumen that is too small may not adequately unload the LA and relieve symptoms; a lumen that is too large may overload the RA and right-heart more generally, creating new problems for the patient. Moreover, the relationship between pressure reduction and clinical outcomes and the degree of pressure reduction required for optimized outcomes is still not fully understood, in part because the pathophysiology for HFpEF (and to a lesser extent, HFrEF) is not completely understood. As such, clinicians are forced to take a best guess at selecting the appropriately sized shunt (based on limited clinical evidence) and generally cannot adjust the sizing over time. Worse, clinicians must select the size of the shunt based on general factors (e.g., the size of the patient's anatomical structures, the patient's hemodynamic measurements taken at one snapshot in time, etc.) and/or the design of available devices rather than the individual patient's health and anticipated response. With many such traditional devices, the clinician does not have the ability to adjust or titrate the therapy once the device is implanted, for example, in response to changing patient conditions such as progression of disease. By contrast, interatrial shunting systems configured in accordance with embodiments of the present technology allow a clinician to select the size-perioperatively or post-implant-based on the patient.

A further challenge with conventional interatrial shunts is that the function of the LA (and more generally, the cardiovascular system) can vary depending on a number of factors, for example during exercise, during periods where a patient's medication adherence has slipped, as the patient's disease progresses, or during other periods. Existing conventional shunts are generally static in nature and lack an ability to adapt to patient conditions in such a way to optimize therapy.

Other shortcomings of existing conventional interatrial shunts include: (1) shunts tending to be permanently implanted in the septal wall in a way that complicates or prevents future transseptal access, which may prohibit or complicate additional left-heart procedures that generally would require transseptal access; (2) shunts tending to be fixed and unable to adapt to changing patient conditions, such as progression of disease, and (3) a lack of sensors and/or machine-learning capability that limit the information available from the patient and limit the ability to improve therapy for the patient (or for the larger patient cohort) over time.

### B. Select Embodiments of Intracardiac Pressure Monitoring Devices

The present technology is generally directed to devices for monitoring intracardiac pressures. These devices may be valuable as stand-alone devices or for use in an interatrial shunting system or other implanted cardiovascular treatment system. Such devices can include a clip structure configured to secure the device to a site within the patient's heart (e.g., to the septal wall). For example, the clip structure can apply a compressive force against the septal wall to anchor the device thereto. The clip structure can be coupled to one or more pressure sensors for monitoring pressure within one or more heart chambers (e.g., the LA and/or RA). In embodiments where the clip structure includes multiple pressure sensors, each pressure sensor can be coupled to a different portion of the clip structure, such that different pressure sensors can measure pressures of different portions of the heart. For example, the device can include a first pressure sensor configured to measure left atrial pressure and/or a second pressure sensor configured to measure right atrial pressure. In other embodiments, multiple pressure sensors are co-located near the same portion of the clip structure, but include or are operably coupled to a pressure transmission element (e.g., a fluid-filled tube) configured to measure pressures at different portions of the heart. For example, the device can include a first pressure transmission element positioned at least partially within the LA and operably coupled to a first pressure sensor, and a second pressure transmission element positioned at least partially within the RA and operably coupled to a second pressure sensor. In further embodiments, one or more pressure sensors may not be mechanically-coupled to the clip structure. In such embodiments, a pressure sensor can be operably coupled to the clip structure and/or to a component that is mechanically-coupled to the clip structure. The pressure measurements can be used to selectively adjust an implanted interatrial shunting element, as described in detail below. The embodiments described herein are expected to improve pressure sensor performance and reduce variability in pressure measurements (e.g., due to motion of the heart).

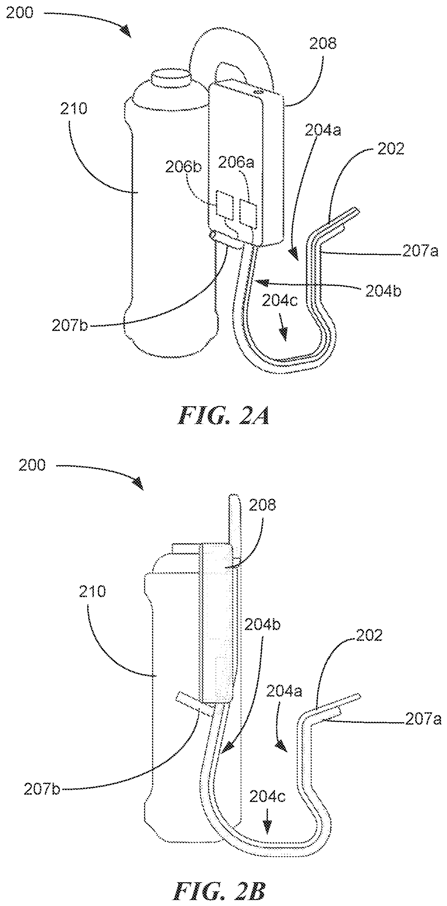

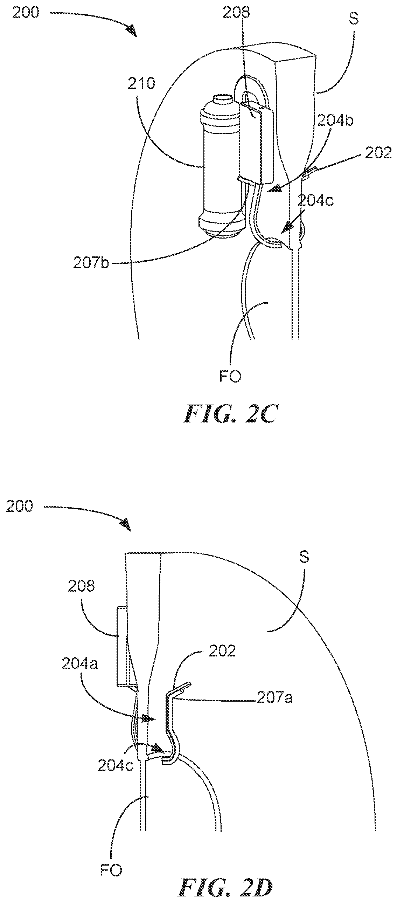

FIGS. 2A-2D illustrate an intracardiac pressure monitoring device 200 configured in accordance with an embodiment of the present technology. More specifically, FIGS. 2A and 2B are perspective and side views, respectively, of the device 200; FIG. 2C is a cross-sectional perspective view of the implanted device 200 from the right side of the septal wall S; and FIG. 2D is a cross-sectional perspective view of the implanted device 200 from the left side of the septal wall S.

Referring to FIGS. 2A-2D together, the device 200 includes a clip structure 202 configured to secure the device 200 to a portion of the patient's heart. In some embodiments, for example, the clip structure 202 is configured to anchor the device 200 to the septal wall S between the LA and the RA, e.g., at or near the fossa ovalis FO (as shown in FIGS. 2C and 2D). For example, when the device 200 is implanted, the clip structure 202 can pierce through the outer edge portion of the fossa ovalis FO and anchor onto surrounding tissue of the septal wall S (e.g., the secundum) located away from the center of the fossa ovalis FO. This configuration can accommodate other transseptal or septal procedures accessing the fossa ovalis FO, such as a procedure to implant an interatrial shunting system as described in greater detail below.

In some embodiments, the clip structure 202 is an elongated structure having a generally bent and/or U-shaped configuration including a first clip segment 204a, a second clip segment 204b, and a hinge segment 204c therebetween connecting the first and second clip segments 204a-b. In embodiments in which the clip structure 202 is an elongated structure (e.g., a wire-like structure, a tubular structure, etc.), the structure can be configured to maintain a predetermined shape/arrangement upon deployment as described in greater detail below. The first and second clip segments 204a-b can be generally parallel to each other, while the hinge segment 204c can be generally orthogonal to the first and second clip segments 204a-b. Each of the segments 204a-c can be linear, curved, or can include one or more linear portions and one or more curved portions. In the illustrated embodiment, each of the segments 204a-c has a different length (e.g., the second clip segment 204b is longer than the first clip segment 204a, and the first clip segment 204a is longer than the hinge segment 204c). In other embodiments, two or more of the segments 204a-c can have the same length.

As best seen in FIGS. 2C and 2D, the clip structure 202 can be configured to engage a portion of the septal wall S to anchor the device 200 thereto. For example, the spacing between the first and second clip segments 204a-b can be sufficiently large to receive a portion of the septal wall S therein. When the clip structure 202 is implanted in the patient's heart, the first clip segment 204a can engage a first side of the septal wall S (e.g., the left atrial side), the second clip segment 204b can engage a second, opposite side of the septal wall S (e.g., the right atrial side), and the hinge segment 204c can traverse the septal wall S (e.g., through an aperture formed therein). In some embodiments, the clip structure 202 is made from an elastic and/or spring-like material (e.g., nitinol manufactured to exhibit superelastic properties at body temperature) such that the first and second clip segments 204a-b are biased towards each other and maintain a desired shape/arrangement upon deployment. As a result, when the clip structure 202 is implanted as shown in FIGS. 2C and 2D, the first and second clip segments 204a-b can apply a force (e.g., a compressive force) against the septal wall S to anchor the device 200 in place. The applied force can be selected to allow for effective anchoring while avoiding inadvertent tissue damage. For example, the force can be large enough to prevent the device 200 from becoming dislodged, e.g., by contractile motions of the heart, but also not so large as to perforate or otherwise damage the tissue. Optionally, the device 200 can also include one or more anchoring elements (e.g., hooks, barbs, etc.-not shown) coupled to and/or formed in the clip structure 202 to secure the device 200 to the septal wall S. In some embodiments, the device 200 can also include one or more magnetic elements (e.g., coupled to the first and second clip segments 204a-b) that provide a compressive force against the tissue of the septal wall S once the device 200 is implanted. In some embodiments the device 200 can be coupled to and/or augmented by additional fixation elements (e.g., a suture, an adhesive, etc.) to improve stability of device 200 once implanted. As described in further detail below, some embodiments of the device 200 can include additional features and/or aspects of the clip structure 202 to improve the stability of device 200 once implanted.

Referring again to FIG. 2A, the device 200 further includes one or more pressure sensors (e.g., a first pressure sensor 206a and a second pressure sensor 206b-not shown in FIGS. 2B-2D merely for purposes for clarity). Many different types of pressure sensors are suitable for use in the device 200 (e.g., piezoresistive, piezoelectric, capacitive, electromagnetic, strain-based, optical, etc.). The pressure sensor(s) can be configured to measure intracardiac pressure when the device 200 is implanted in the patient's heart. For example, the first pressure sensor 206a can be operably coupled to a first pressure transmission element 207a (e.g., a first fluid-filled tube) and the second pressure sensor 206b can be operably coupled to a second pressure transmission element 207b (e.g., a second fluid-filled tube). The first and second pressure transmission elements 207a-b can each be configured to transmit pressure from a corresponding region of the heart to the first and second pressure sensors 206a-b, respectively. Alternatively or additionally, a pressure sensor can be placed inside of a cavity or well (e.g., a cavity created in the clip structure or in a component carried by the clip structure) that is covered by a thin, stiff membrane or diaphragm (e.g., a titanium diaphragm). The cavity or well may be filled with an incompressible fluid (e.g., an oil) that transmits pressures experienced by the diaphragm efficiently to the pressure sensor. In some embodiments, at least one of the first and second pressure transmission elements 207a-b are positioned at least partially in the portions of the heart where pressure measurements are to be taken (e.g., the LA and RA), while the first and second pressure sensors 206a-b are at a different location (e.g., within a housing of a controller 208 of the device 200). For example, referring to FIGS. 2C and 2D together, the first pressure transmission element 207a can be carried by and/or coupled to the first clip segment 204a such that one or more portions of the first pressure transmission element 207a are positioned within the LA when the device 200 is implanted. The second pressure transmission element 207b can be carried by and/or coupled to the second clip segment 204b such that one or more portions of the second pressure transmission element 207b are positioned within the RA when the device 200 is implanted. In some embodiments, two or more pressure sensors may be configured differently from one another. For example, in some implementations of the device 200, a first sensor may be configured to measure pressure in a region directly, while a second sensor may be configured to measure pressure through the use of a pressure transmission element.

In the illustrated embodiment, the first and second pressure transmission elements 207a-b are each configured as an elongated tube filled with a fluid (e.g., water, silicone oil, etc.). The individual elongated tubes can have a diameter less than or equal to 1 mm, 2 mm, 3 mm, 4 mm, or greater than 4 mm. The shape of the elongated tubes can be generally similar to the shape of the corresponding portion(s) of the clip structure 202 to which the elongated tube is coupled. For example, in the illustrated embodiment, the elongated tube of the first pressure transmission element 207a can have a shape generally similar to the shape of the first clip segment 204a and/or hinge segment 204c. In other embodiments, the shape of the elongated tube(s) can be different or dissimilar to the shape of the corresponding portion(s) of the clip structure 202 to which the elongated tube is coupled, which can result in different directionality and/or positioning of sections of tube and clip structure (e.g., diverging end portions).

The end surface of each elongated tube can be covered with a pressure-responsive membrane. The end surface can be located within a region of the heart (e.g., the LA or RA), such that the pressure within the heart region is transmitted to the fluid within the elongated tube via the pressure-responsive membrane. The elongated tube can be operably coupled to a pressure sensor (e.g., first and/or second pressure sensors 206a-b) so that the pressure sensor measures the pressure of the fluid within the elongated tube to generate a pressure measurement for the heart region. In some embodiments, one or more portions of the elongated tube extend away from the septal wall S when implanted, such that the end surface is positioned at least 1 mm, 2 mm, 3 mm, 4 mm, 5 mm, 6 mm, 7 mm, 8 mm, 9 mm, 10 mm, or more away from the septal wall S. This configuration is expected to reduce, delay, or prevent endothelialization or other tissue growth over the pressure-responsive membrane that can interfere with measurement accuracy.

The embodiment shown in FIG. 2 is expected to provide a number of advantages over conventional devices because the present technology enables bi-atrial pressure sensing while allowing for only a small portion of the device to reside inside of the LA. In general, it is considered clinically-advantageous to minimize the amount of foreign material implanted into the LA to minimize or inhibit the risk of thrombus formation. The integration of a pressure transmission element into the device enables an LA signal to be measured directly while keeping the larger structures associated with the pressure sensor and related housing and electronics in the RA. Further, the use of a pressure transmission element similar to that shown in FIG. 2 is expected to minimize the size of the transseptal aperture that needs to be created during device implantation. Such a reduction in size of the septal aperture created during implantation is expected to improve the safety of the procedure, and is believed to lead to more rapid healing (i.e., closing of the aperture to isolate the atria) of the septal wall.

The device 200 can further include a controller 208 coupled to the clip structure 202. In the illustrated embodiment, the controller 208 is coupled to the second clip segment 204b such that when the device 200 is implanted, the controller 208 is positioned against the right atrial side of the septal wall S (e.g., as shown in FIG. 2C). In other embodiments, the controller 208 can be coupled to other portions of the clip structure 202, such as the first clip segment 204a. In such embodiments, the controller 208 can be positioned against the left atrial side of the septal wall S when the device 200 is implanted. The controller 208 can be used to house various electronic components of the device 200, such as processors, memory, actuation mechanisms, sensors, communication devices, etc. In some embodiments, the controller 208 may be separate from and/or unattached to the clip structure 202, but may be operably coupled to and/or in communication with the clip structure 202 and/or components integral with or attached to the clip structure 202 (e.g., via a wireless signal transmission).

In some embodiments, the first and second pressure sensors 206a-b are located within the controller 208 and are operably coupled to the first and second pressure transmission elements 207a-b to generate intracardiac pressure data, as previously described herein. The controller 208 can store the pressure data, and can optionally process the pressure data (e.g., to calculate a pressure differential between the LA and the RA, calculate changes in pressure over time, etc.). Optionally, the controller 208 can use an antenna (not shown) or other communication device to transmit the pressure data to a device external to the patient, such as to a mobile device or other computing device operated by a user (e.g., a clinician). In some embodiments, the controller 208 is configured to store measured pressure data (e.g., one or more pressure measurements, multiple pressure measurements taken over a period of time, etc.) without the patient interfacing with an external device.

The device 200 can further include one or more energy storage components (e.g., energy storage component 210) coupled to or otherwise operably integrated with the clip structure 202. In the illustrated embodiment, the energy storage component 210 is coupled to the second clip segment 204b such that when the device 200 is implanted, the energy storage component 210 is positioned against the right atrial side of the septal wall S (e.g., as shown in FIG. 2C). In other embodiments, the energy storage component 210 can be coupled to other portions of the clip structure 202, such as the first clip segment 204a. In such embodiments, the energy storage component 210 can be positioned against the left atrial side of the septal wall S when the device 200 is implanted. In some embodiments, the device 200 can include a plurality of energy storage components that are positioned in multiple anatomical regions (e.g., in both the LA and RA, against both the LA and RA septal walls, etc.)

The energy storage component 210 can be a primary battery, a secondary battery (e.g., a rechargeable battery), a supercapacitor, a capacitor, or another suitable element capable of retaining energy. The energy storage component 210 can be operably coupled to one or more components of the device 200 to provide power thereto. For example, the energy storage component can be used to power the pressure sensors 206a-b and/or the controller 208.

The device 200 can be configured in many different ways. For example, although FIGS. 2A-2D depict the device 200 as having two pressure sensors 206a-b, in other embodiments the device 200 can include a different number of pressure sensors (e.g., one, three, four, five, or more). In embodiments where the device 200 includes multiple pressure sensors, the sensors can be of different types (e.g., one piezoelectric sensor, one capacitive sensor, one optical sensor, etc.). Optionally, the first pressure sensor 206a or the second pressure sensor 206b can be omitted, such that the device 200 is used to measure pressure within a single heart chamber (e.g., the LA). Moreover, in other embodiments the first and second pressure transmission elements 207a-b can be carried by and/or coupled to portions of the device 200 other than the first and second clip segments 204a-b (e.g., the hinge segment 204c). Additionally, the first and second pressure sensors 206a-b can be located outside the controller 208 (e.g., at or near a portion of the clip structure 202) and can be operably coupled to the controller 208 (e.g., via a wired or wireless communication) to allow for processing and/or storage of generated pressure data. The integration of more than two pressure sensors can be useful in a number of ways. For example, multiple sensors can allow a device to measure pressure in more locations (e.g., in more anatomical regions, in different areas of a single anatomical region, etc.) or measure/estimate the flow of fluid between anatomical regions of areas of an anatomical region. Additionally, multiple sensors may allow for improved accuracy and/or enable a device to facilitate calibration of the sensors, reduce measurement artifacts, reduce and/or account for sensor drift, and other operational benefits.

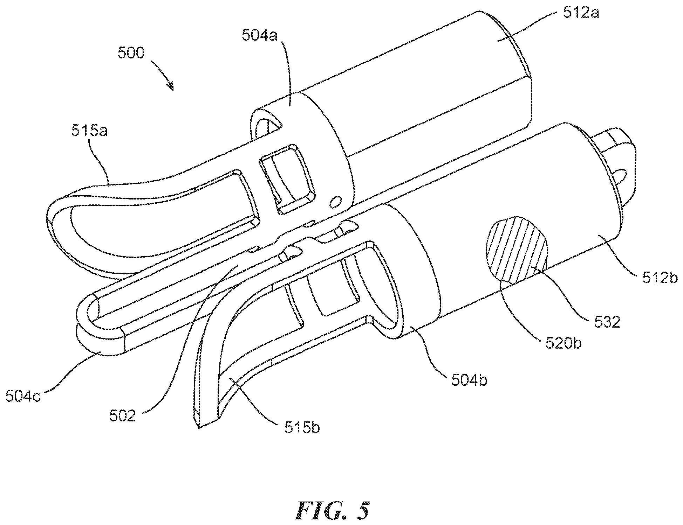

In some embodiments, the first and second pressure transmission elements 207a-b are omitted such that the pressure sensors 206a-b are configured to measure intracardiac pressure directly without any intervening components to transmit pressure thereto. In such embodiments, each pressure sensor 206a-b can be positioned within a region of the heart to directly measure the pressure therein. For example, the first pressure sensor 206a can be carried by the first clip segment 204a and positioned within the LA to measure left atrial pressure, and the second pressure sensor 206b can be carried by the second clip segment 204b and positioned with the RA to measure right atrial pressure. The first and second pressure sensors 206a-b can be operably coupled to the controller 208 (e.g., via wired or wireless communication) to allow for processing and/or storage of generated pressure data. In further embodiments, the device 200 can include alternative pressure transmission elements 207 that do not comprise an elongated tube or similar structure. In such embodiments (such as the embodiment described below with reference to FIGS. 5-6C), pressure sensor(s) may reside in a relatively shallow well or cavity that is covered by a thin pressure sensitive membrane or diaphragm. The cavity can be filled with a fluid to improve the effectiveness of pressure transmission from the diaphragm to the pressure sensor(s).

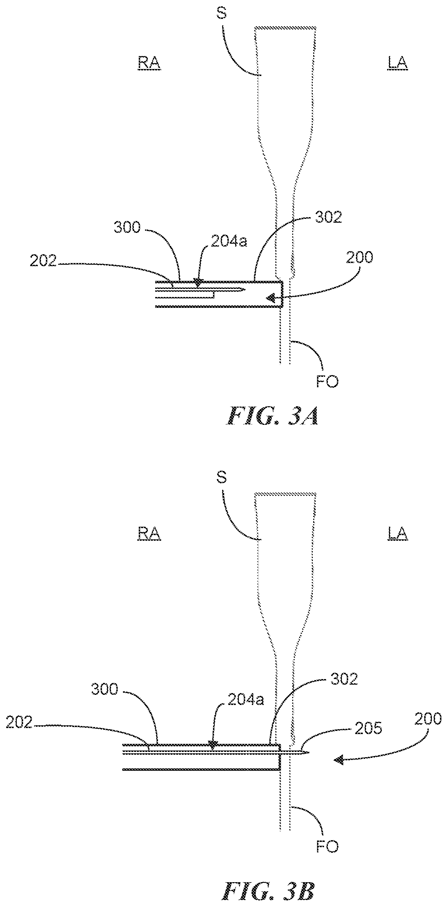

FIGS. 3A-3D are side cross-sectional views of the intracardiac pressure monitoring device 200 being implanted in a patient's heart in accordance with an embodiment of the present technology. In some embodiments, the device 200 is configured to transform from a delivery configuration to an operating configuration during the implantation procedure. When in the delivery configuration, one or more components of the device (e.g., the clip structure 202 and/or pressure transmission elements 207a-b) can each have a low-profile shape (e.g., a generally flattened, and/or contracted shape) to facilitate introduction into the heart (e.g., via a percutaneous route). For example, when in the delivery configuration, the clip structure 202 and/or pressure transmission elements 207a-b can be sufficiently flattened or otherwise contracted to fit within a delivery catheter. Once the device is positioned at or near the implantation site, the clip structure 202 and/or pressure transmission elements 207a-b can be transformed into the operating configuration. When in the operating configuration, the clip structure 202 can have a generally bent and/or curved shape configured to couple to the septal wall S, and the pressure transmission elements 207a-b can be positioned away from the septal wall S (e.g., as previously described with respect to FIGS. 2A-2D). The clip structure 202 can be flexible in nature to accommodate variations in septal wall thickness (both thickness variation along the septal wall of a particular patient and variations in thickness of the septal wall between patients).

Various techniques can be used to selectively transform/deploy the clip structure 202 and/or pressure transmission elements 207a-b between the delivery configuration and the operating configuration. For example, the clip structure 202 and/or pressure transmission elements 207a-b can each be made from a superelastic material, such as nitinol. In some embodiments, the clip structure 202 and/or pressure transmission elements 207a-b are shape set into the operating configuration in accordance with techniques known to those of skill in the art. Subsequently, the clip structure 202 and/or pressure transmission elements 207a-b can be transformed into the delivery configuration, e.g., by mechanical loading. When the mechanical loading is removed, the clip structure 202 and/or pressure transmission elements 207a-b can transform between the delivery configuration and the operating configuration due to the superelastic effect.

Referring first to FIG. 3A, the device 200 is positioned within a delivery catheter 300 in a delivery configuration. When in the delivery configuration, the clip structure 202 can have a low-profile shape (e.g., a generally flattened, and/or contracted shape), rather than the generally bent and/or U-shaped configuration previously described with respect to FIGS. 2A-2D. Optionally, the pressure transmission elements 207a-b can also be in a low-profile delivery configuration. In some embodiments, the clip structure 202 and/or pressure transmission elements 207a-b are constrained in the delivery configuration by the interior surfaces of the delivery catheter 300. The delivery catheter 300 can apply mechanical loading to the clip structure 202 and/or pressure transmission elements 207a-b to prevent these components from reverting to the operating configuration.

The device 200 can be introduced into the patient's heart via the delivery catheter 300. The delivery catheter 300 can be advanced to a position adjacent or near the septal wall S (e.g., adjacent or near the fossa ovalis FO). In some embodiments, the delivery catheter 300 is advanced through the right atrium RA and positioned against the right atrial side of the septal wall S. To avoid injury or damage to the device, the device 200 can remain sheathed within the delivery catheter 300 such that the first clip segment 204a of the clip structure 202 does not protrude past the distal end portion 302 of the delivery catheter 300, e.g., until the delivery catheter 300 is positioned against the septal wall S.

Referring next to FIG. 3B, the delivery catheter 300 can remain in place while the device 200 is advanced distally through the delivery catheter 300 and towards the septal wall S. As the device 200 is advanced, the first clip segment 204a can protrude out of the distal end portion 302 and contact, traverse, or otherwise interface with the septal wall S. The first clip segment 204a can include a sharpened and/or tapered end portion 205 configured to pierce through or otherwise facilitate passage across the septal wall S (e.g., through the fossa ovalis FO). In some embodiments, the catheter 300 or another aspect of the device or delivery system may be used to form an aperture through the septal wall S. In other embodiments, a separate tool or a separate component of the device 200 and/or delivery catheter 300 is used to create a transseptal puncture to form the aperture prior to advancing the device 200 towards the septal wall S.

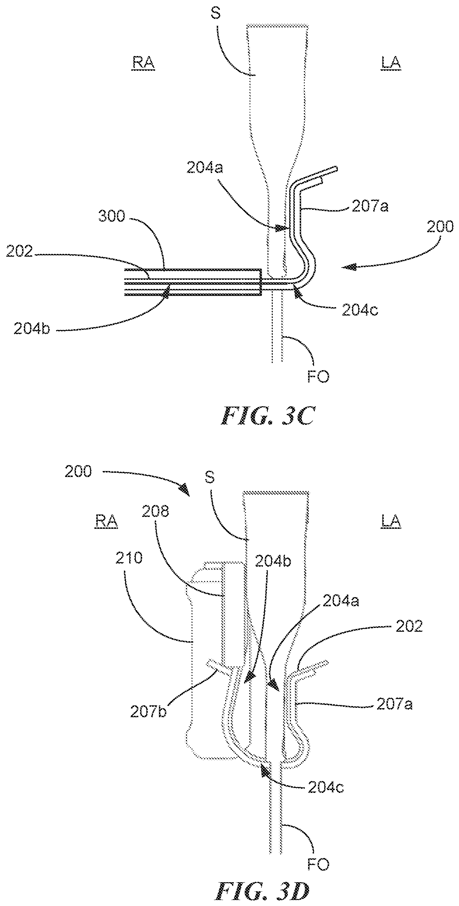

Referring next to FIG. 3C, the first clip segment 204a and the first pressure transmission element 207a can be pushed through the aperture in the septal wall S and into the left atrium LA. As the device 200 continues to advance distally relative to the delivery catheter 300 (e.g., by retracting the catheter 300 or a sheath while holding the device position stable in the LA, by pushing the device out of the catheter 300 while holding the catheter position stable in the LA, etc.), the clip structure 202 can progressively transform to the operating configuration (e.g., due to a superelastic effect as described above). With the first clip segment 204a deployed, the catheter 300 can be retracted back through the FO and into the RA (as shown in FIG. 3C), resulting in the deployed portion of device 200 being pulled into position along the LA side of the septal wall. Portions of the clip structure 202 that are still constrained within the delivery catheter 300 can remain in the delivery configuration, while portions of the clip structure 202 that are outside of the delivery catheter 300 and therefore no longer subject to mechanical loading by the delivery catheter 300 can transform to the operating configuration. For example, in the illustrated embodiment, the first clip segment 204a is entirely outside of the delivery catheter 300 and has transformed into a bent and/or curved shape engaging the left atrial side of the septal wall S. The hinge segment 204c is partially outside the delivery catheter 300, and therefore has only partially transformed into a bent and/or curved shape. The second clip segment 204b is entirely within the delivery catheter 300 and therefore remains in a low-profile shape.

In embodiments where the first pressure transmission element 207a is also made from a superelastic material such as nitinol, the first pressure transmission element 207a can also transform from a delivery configuration to an operating configuration as it exits the delivery catheter 300. When in the operating configuration, the first pressure transmission element 207a can extend away from the septal wall S, as previously described. In other embodiments the first pressure transmission element 207a can be made of a deformable material that does not exhibit superelastic properties, or can otherwise be constructed such that it can be bendable and/or deflectable. In such embodiments, the first pressure transmission element 207a can be coupled to the first clip segment 204a such that as the first clip segment 204a transforms to the operating configuration, it applies a force to the first pressure transmission element 207a that transforms the first pressure transmission element 207a into the operating configuration as well. In some embodiments, a suitable material for a pressure transmission element 207a can allow for ideal positioning as it deploys into the operating configuration as described above while also maintaining a sufficiently rigid wall strength so as to not introduce losses in the pressure signal as it is conveyed to the corresponding sensor.

Referring next to FIG. 3D, the delivery catheter 300 can be retracted away from the septal wall S to fully deploy the device 200. As a result, the entirety of the clip structure 202 can transform to the operating configuration to secure the device to the septal wall S, as previously described. For example, in the illustrated embodiment, the second clip segment 204b has also transformed to a bent shape engaging the right atrial side of the septal wall S. The controller 208 and energy storage component 210 can also be deployed out of the delivery catheter 300 and folded up against and/or near the right atrial side of the septal wall S. In some embodiments, the controller 208 and energy storage component 210 are coupled to and/or carried by the second clip segment 204b such that the transformation of the second clip segment 204b (via the superelastic effect described above) also positions the controller 208 and energy storage component 210 into the desired configuration upon release from the delivery catheter 300.

In embodiments where the second pressure transmission element 207b is also made from a superelastic material such as nitinol, the second pressure transmission element 207b can also transform from a delivery configuration to an operating configuration as it exits the delivery catheter 300 due to the superelastic effect. When in the operating configuration, the second pressure transmission element 207b can extend away from the septal wall S, as previously described. In other embodiments, the second pressure transmission element 207b can be made of a deformable material that does not exhibit superelastic properties or can otherwise be constructed such that it can be bendable and/or deflectable. In such embodiments, the second pressure transmission element 207b can be coupled to the second clip segment 204b such that as the second clip segment 204b transforms to the operating configuration, it applies a force to the second pressure transmission element 207b that transforms the second pressure transmission element 207b into the operating configuration as well.

Although not shown in FIGS. 3A-3D, some embodiments of the devices and delivery methods described herein use a guidewire for delivery such that the device 200 is configured to be delivered using "over the wire" techniques known to those of skill in the art. One advantage of over the wire delivery methods is that devices that are maldeployed or otherwise in an undesired location (e.g., the device is embolized entirely in the left atrium) can be easier to retrieve safely. In other embodiments, instead of using over the wire delivery techniques, the device 200 may be connected to a delivery catheter with releasable tethers that a user can detach/remove once confident that the device has been delivered properly.

Moreover, in other embodiments the transformation of the device 200 between the delivery configuration and the operating configuration can be actuated by a shape memory effect instead of or in addition to a superelastic effect. In such embodiments, the clip structure 202 and/or pressure transmission elements 207a-b can each be made from a shape memory material. The shape memory material can include a shape memory metal or alloy (e.g., nitinol that has been manufactured and/or treated appropriately as understood by those skilled in the art), a shape memory polymer, a pH-based shape memory material, or any other suitable material configured to change in shape (e.g., transform between a first configuration and second configuration) in response to a stimulus (e.g., heat or mechanical loading). The clip structure 202 and/or pressure transmission elements 207a-b can be initially shape set into the operating configuration and subsequently transformed into the delivery configuration to facilitate implantation in the patient's heart. When the device 200 has been positioned at the appropriate location, the clip structure 202 and/or pressure transmission elements 207a-b can be transformed back into the operating configuration by application of energy (e.g., heat). In some embodiments, this heat could be applied via normal body temperature that the implant would experience after it is implanted into a body. In such embodiments, an implanted device could be pre-cooled such that it is delivered to the body at a temperature lower than typical room temperature.

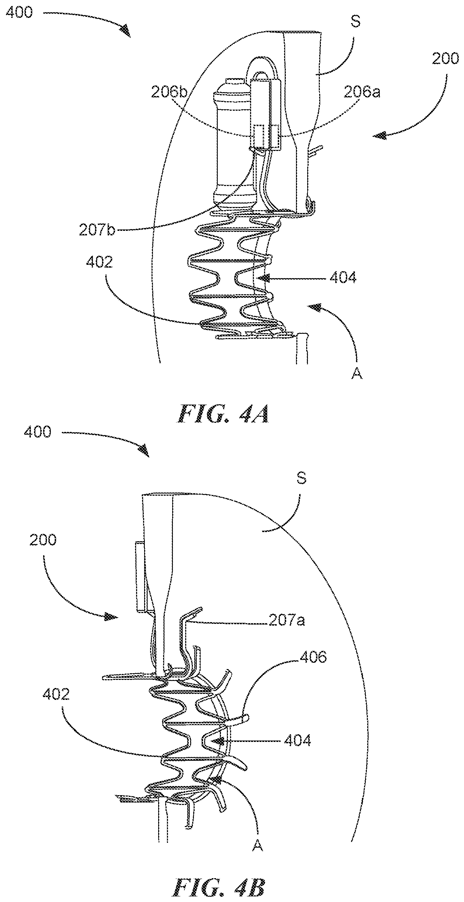

FIGS. 4A and 4B illustrate an interatrial shunting system 400 including the intracardiac pressure monitoring device 200 and configured in accordance with an embodiment of the present technology. More specifically, FIG. 4A is a cross-sectional perspective view of the system 400 from the right side of the septal wall S, and FIG. 4B is a cross-sectional perspective view of the system 400 from the left side of the septal wall S. The system 400 includes a shunting element 402 (e.g., a stent) defining a lumen 404 therethrough. When implanted in the septal wall S, the system 400 fluidly connects the left atrium and the right atrium via the lumen 404. The shunting element 402 can be secured to the septal wall S by anchoring elements 406.

The system 400 further includes the intracardiac pressure monitoring device 200, which can be configured and implanted as previously described with respect to FIGS. 2A-3D. The positioning of the device 200 relative to the shunting element 402 can be selected to reduce or avoid interference with the pressure transmission elements 207a-b and/or to reduce or avoid obstruction of the lumen 404. For example, in the illustrated embodiment, the shunting element 402 is positioned in the septal wall S in an aperture A formed in or near the fossa ovalis (not shown), and the device 200 is positioned in the portion of the septal wall S adjacent or near the aperture A.

The intracardiac pressure monitoring device 200 can be implanted in the patient's heart prior to, concurrently with, or after implantation of the shunting element 402. For example, in some embodiments, the device 200 is implanted first and is used to monitor intracardiac pressure for a period of time (e.g., days, weeks, months, etc.) to determine whether the patient would benefit from an interatrial shunt. If the clinician determines that an interatrial shunt would be beneficial, the shunting element 402 can subsequently be implanted. Optionally, the pressure data generated by the device 200 can be used to determine other treatment parameters, such as the geometry (e.g., size and/or shape) of the shunting element 402. After implantation of the shunting element 402, the device 200 can be used to continue monitoring intracardiac pressure, e.g., to assess performance of the shunting element 402, to determine whether adjustments to the shunting element 402 would be beneficial, etc. In some embodiments the shunting element 402 can be implanted in a closed configuration (i.e., little to no aperture for shunting is available despite the infrastructure for a shunting element being present). In such embodiments the shunting element can be opened (i.e., converted into an active shunt by widening the aperture) at a time following implantation. In some implementations, the decision to convert the shunt from an inactive/closed to an active/open state can be based on data provided by device 200.

In some embodiments, the shunting element 402 is selectively adjustable after implantation to control blood flow between the LA and RA based on pressure data generated by one or more of the pressure sensors 206a-b of the device 200 and/or based upon other data or attributes of the patient's health. For example, the pressure sensors 206a-b can be used to measure pressure in one or more heart chambers over time, and adjustments to the shunting element 402 can be made in response to changes in the measured pressure (e.g., increasing or decreasing pressure over time). As another example, pressure measurements from the pressure sensors 206a-b can be used to calculate a pressure differential between different heart chambers (e.g., the LA and the RA), and adjustments to the shunting element 402 can be made in response to the calculated pressure differential (e.g., whether the pressure differential is greater than or less than a predetermined threshold, whether the pressure differential exceeds a predetermined range, etc.).

In some embodiments, the system 400 includes a flow control mechanism (not shown) configured to adjust the shunting element 402 (e.g., in a non-invasive manner). The flow control mechanism can change a shape or other characteristic of the shunting element 402 to change the flow of fluid through the lumen 404. In some embodiments, the flow control mechanism selectively changes the geometry (e.g., size and/or shape) of the lumen 404 to change the flow resistance through the lumen 404. For example, the flow control mechanism can be configured to selectively increase a diameter of the lumen 404 and/or selectively decrease a diameter of the lumen 404.

Alternatively or in combination, the shunting element 402 can be adjusted by a clinician via a suitable adjustment device. For example, in some embodiments, the shunting element 402 is a balloon-expandable stent having an adjustable lumen diameter (e.g., adjustable within a range from 5 mm to 12 mm). If the clinician determines that an adjustment would be beneficial (e.g., considering data generated by one or more pressure sensors, if the patient's disease progresses, if the shunting element 402 narrows or otherwise becomes obstructed), the clinician can use a balloon catheter or similar adjustment device to adjust (e.g., expand or contract) the diameter of the lumen 404 of the shunting element 402. In some embodiments, the shunting element 402 and/or adjustment device include a radiopaque material, such that the adjustment procedure can be performed under fluoroscopic guidance.

In some embodiments, the size and/or shape of the lumen 404 can be adjusted on a consistent time schedule (e.g., continuously, hourly, daily, monthly, yearly, etc.). Consistent adjustments might be made, for example, to adjust the flow of blood based on an exertion level and/or heart rate of the patient, which changes frequently over the course of a day. For example, the system 400 can have a baseline state in which the lumen 404 is substantially closed and does not allow substantial blood flow between the LA and RA, and an active state in which the lumen 404 is open and allows blood to flow between the LA and RA. The system 400 can transform from the baseline state to the active state whenever the exertion level and/or heart rate of the patient increases due to exercise, stress, or other factors. As another example, consistent adjustments can be made based on, or in response to, sensed physiological parameters, including, for example, sensed left atrial pressure and/or right atrial pressure via pressure sensors 206a-b. If the left atrial pressure increases, the system 400 can automatically increase a diameter of the lumen 404 to increase blood flow between the LA and the RA. In yet another example, the system 400 can be configured to adjust based on, or in response to, an input parameter from another device such as a pulmonary arterial pressure sensor, insertable cardiac monitor, pacemaker, defibrillator, cardioverter, wearable, external ECG or PPG, and the like.

Some embodiments of the present technology adjust the relative size and/or shape of the lumen 404 only after a threshold has been reached (e.g., a sufficient period of time has elapsed). This may be done, for example, to avoid unnecessary back and forth adjustments and/or avoid changes based on clinically insignificant changes in patient condition. In some embodiments, adjustments may occur occasionally as a patient's condition changes. For example, the lumen 404 may gradually open if a patient experiences a sustained rise in left atrial pressure (e.g., rate of change is above a predetermined threshold, and/or the left atrial pressure remains higher than a predetermined threshold for longer than a predetermined amount of time), pulmonary artery pressure, weight, or another physiologically relevant parameter. Alternatively or in combination, adjustments can occur if pressure exceeds a threshold or increases by a threshold amount over a period of time (e.g., several days or more). The diameter of the lumen 404 can then be increased to increase blood flow between the LA and RA and to avoid decompensation.

The system 400 can also enable a clinician to periodically (e.g., monthly, bimonthly, annually, as needed, etc.) adjust the diameter of the lumen 404 to improve patient outcomes. For example, during a patient visit, the clinician can assess a number of patient parameters and determine whether adjusting the diameter of the lumen 404, and thus altering blood flow between the LA and the RA, would provide better treatment and/or enhance the patient's quality of life. Patient parameters can include, for example, physiological parameters (e.g., left atrial blood pressure, right atrial blood pressure, the difference between left and right atrial blood pressures, flow velocity, heart rate, cardiac output, myocardial strain, etc.), subjective parameters (e.g., whether the patient is fatigued, how the patient feels during exercise, etc.), and other parameters known in the art for assessing whether a treatment for HFpEF is working.

Some embodiments of the devices disclosed herein include additional features to provide increased positional stability of the clip structure and other device structures both during and following implantation into the atrial septal wall. For example, in view of the taper in thickness between the secundum and primum regions of the septum, as well as the low friction surface of the septal myocardium, there may exist circumstances (e.g., in a patient with a thickened secundum region) in which a simplified clip design is not the optimal choice from a positional stability viewpoint. Further, in some patients (e.g., those with abnormally thin septal walls) it may be possible for a hinge segment of a clip structure (e.g., hinge segment 204c-FIGS. 2C and 2D) to slide into one of the atria instead of engaging with the septal wall following deployment from a delivery catheter. In both scenarios described above, it may be difficult for a physician to reliably obtain the desired implantation position of the device and, in some circumstances, it may be possible for a partially or completely-deployed device (e.g., a device in the operating configuration) to be inadvertently deposited entirely in a single atrial chamber.

FIG. 5 illustrates an intracardiac pressure monitoring device 500 configured in accordance with another embodiment of the present technology. The device 500 includes a clip structure 502 having a first clip segment 504a configured to contact a first surface of a septal wall (not shown), a second clip segment 504b configured to contact the opposing surface of the septal wall, and a hinge segment 504c extending therebetween and configured to traverse the septum. The clip structure 502 also includes first and second flared segments 515a and 515b that originate from the first and second clip segments 504a-b, respectively, and extend toward hinge segment 504c while slightly flaring outward away from the hinge segment 504c (i.e., flaring into the atria). In operation, the flared segments 515a-b are expected to provide the clip structure 502 with enhanced stability during and following deployment onto the septal wall, and are further expected to provide an implanting physician with improved control of the implant during the placement procedure. For example, in some patients, the flared segments 515a-b are expected to reduce the likelihood that the first portion of the device (i.e., the structures generally associated with clip segment 504a that are intended to reside in the LA) is inadvertently retracted into the RA after it deployed from a delivery catheter (not shown) and assumes its operating configuration, even if hinge segment 504c initially lands entirely in either the LA or RA (e.g., in scenarios in which a patient has an abnormally thin fossa ovalis and/or septal wall).

The device 500 also includes first and second canisters 512a-b. The canisters 512 can contain various structures/components (not shown) associated with the operation of the device 500, including energy storage component(s), microcontroller(s) and/or other electronic components, transmission antennae, energy receiving component(s), pressure sensor(s), pressure transmission element(s), and/or other suitable components. In the embodiment shown in FIG. 5, the device 500 includes a first pressure transmission element (not shown) and a second pressure transmission element 520b that are configured as cavities in first and second canisters 512a-b respectively. The first and second pressure transmission elements 520 contain pressure sensors (not shown) operably coupled to a corresponding pressure sensitive diaphragm 532. In some embodiments, each cavity may be filled with a fluid to facilitate the transfer of pressure signals from the diaphragm 532 to the respective sensor(s).

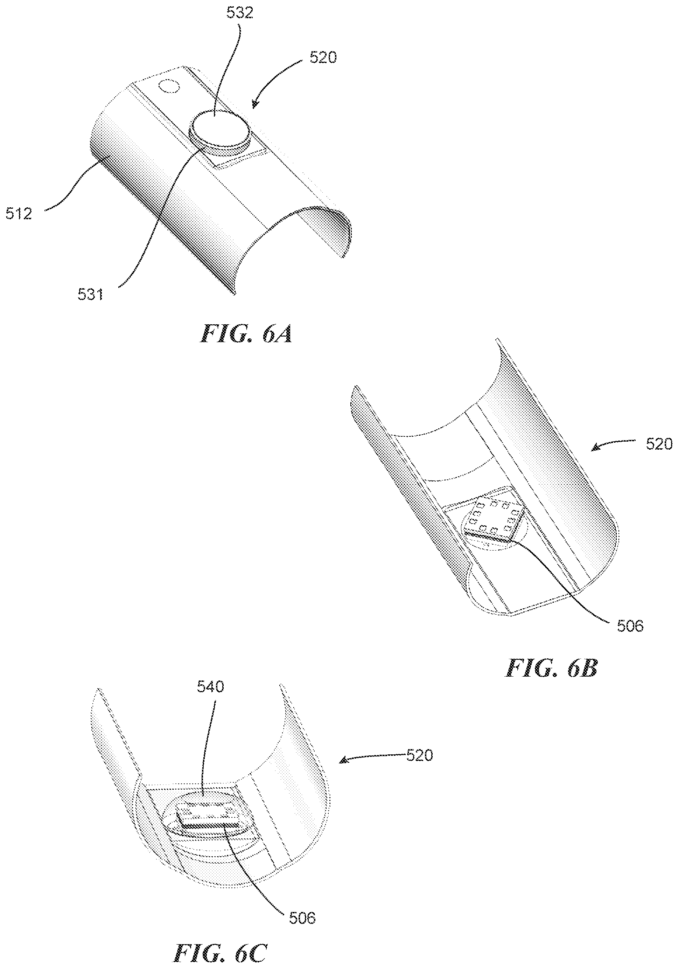

FIGS. 6A-6C illustrate aspects of a manufacturing process for one of the cavity-style pressure transmission elements 520 of FIG. 5 in further detail. More specifically, FIG. 6A is an isometric view of an exterior or first side of the pressure transmission element 520, FIG. 6B is an isometric view of an interior or second side of the pressure transmission element, and FIG. 6C is another isometric view of the bottom or second side of the pressure transmission element at a different stage of the manufacturing process relative to FIGS. 6A and 6B. Referring first to FIG. 6A, a pressure sensitive diaphragm 532 is affixed to an interface element 531 that interfaces with the exterior side of the canister 512. Referring next to FIG. 6B, on the interior side of the canister, a pressure sensor 506 is affixed to the interface element 531 and/or to another structure in the vicinity of the interface element. In some embodiments, additional supporting components including, but not limited to, structural components and electrical components (not shown for clarity), are included in the pressure transmission element 520 to further enhance operation and integration of the sensor 506 with the rest of the device (FIG. 5).

Referring next to FIG. 6C, a cap structure 540 surrounds the sensor 506 and interfaces with and/or surrounds the interface element 531 (FIG. 6A) to complete the cavity/well that serves as the pressure transmission element 520. Referring to FIGS. 6A-6C together, during manufacturing, the cavity defined by the space between the diaphragm 532 and cap structure 540 may be filled with a fluid (not shown) to facilitate transfer of pressure signals from the diaphragm 532 to the sensor 506.

The present embodiment of the pressure transmission element is expected to provide several advantages. For example, by utilizing a cavity/well approach, it is possible to limit the distance between the diaphragm and the sensor. This may lead to fewer measurement artifacts and limit the volume of fluid/oil that is needed to transmit pressure between the diaphragm and the sensor (i.e., it is not necessary to fill an entire canister or entire elongated tube with fluid). Further, in the embodiment illustrated in FIG. 5, the pressure transmission elements 520 are located on the exterior facing sides of canisters 512a-b (i.e., the region of the canisters 512a-b most distant from the septal wall), which is expected to limit the degree of tissue overgrowth that might impact the accuracy of resolution of the device's pressure measurements.

In further embodiments, the clip structure may be comprised of various wireform structures. In one implementation, for example, a device includes a single wire (e.g., a superelastic nitinol wire) shaped to form two loop segments, with each loop segment interfacing with a canister or other component of the device. Upon delivery (e.g., via a delivery catheter), a first loop segment is deployed in the LA and can carry with it a component of the device (e.g., a controller, a pressure sensor, a pressure transmission element, etc.). Similarly, following retraction of the delivery catheter into the RA, a second loop segment (which, in some embodiments, can carry a second component of the device) is deployed. As the device is removed from the delivery catheter, it assumes its operating configuration, with both loop segments (and, accordingly, any device components attached to the loop segments) resting against the septum and exerting a mild squeezing (retention) force on the septal wall. In such a configuration, the loop segments (along with components attached to the loop segments) serve as the vehicle to exert the mechanical forces of the clip structure. Additionally, the generally ovular shape of the loop segments is expected to allow for improved stability of the implanted device by increasing the amount of septum surface area that the clip structure engages, and is further expected to reduce the likelihood of device migration or maldeployment. Further, a wireform clip structure can enable the clip portion of the device to serve additional functions (e.g., to be used as a wireless antenna, to be used as an energy receiving component, or other suitable purposes).

Embodiments of the technology disclosed herein are expected to provide a number of advantages over technologies described in the prior art. For example, some implementations of the present technology allow for bi-atrial pressure sensing to be achieved while leaving little to no device structure in regions proximate to the fossa ovalis. Conventional devices often measure pressure in a single atrial chamber and/or are intended for implantation in the fossa ovalis region. The permanent implantation of devices in the fossa ovalis region can be problematic as it may prohibit or complicate future cardiovascular procedures requiring transseptal access (e.g., pulmonary vein ablation, left atrial appendage closure, mitral valve replacement/repair, etc.). Embodiments of the present technology utilize an efficient clip design that allows for positional stability while minimizing the footprint (i.e., size) of the implant on the septal wall. Further, embodiments of the present technology allow for all of or the bulk of the device structure to reside away from the fossa ovalis (e.g., the embodiments shown in FIG. 2C-2D), thereby providing little to no physical barriers proximate to the fossa ovalis that may complicate or prevent future cardiac procedures.

Clip-type devices and the associated delivery/implantation systems configured in accordance with the present technology are adapted to place such devices in a region of the septal wall remote from the fossa ovalis. The disclosed implantation procedures can involve, for example, puncturing the secundum of the septum to create an aperture through which portions of the device may cross into the LA. Such secundum punctures are rare and large punctures in the thick secundum may be challenging to accomplish and unsafe for the patient. Via the use of small pressure transmission elements attached to clip structures, embodiments of the present technology enable sensing components of the device to be deployed through manageably-sized apertures in the secundum. As such, devices configured in accordance with the present technology are expected to be uniquely suited to be placed remote from the fossa ovalis while retaining the safety, stability, and functionality of the cardiac implant.

In some embodiments of the present technology, the devices described herein may include additional/different features. For example, in some implementations, metallic coils, inductor-based circuits, and/or other circuitry for receiving an external energy signal (e.g., a magnetic energy signal, a radiofrequency signal, etc.) are operably-coupled to the device. In some instances, these coils may receive outside energy and convert that energy to perform tasks such as charging an energy storage device, powering sensor and/or controller operation, facilitating the transfer of data, or other tasks. Some implementations of the device described herein may also include a transmission antenna configured to wirelessly convey data acquired by the sensors to a reader remote from the device (e.g., outside of the body, implanted elsewhere in the body, etc.).

In some embodiments, one or more portions of the disclosed devices (e.g., a clip, a controller, etc.) may be selectively treated with and/or covered by surface texturing, membranes/coatings, or other means to facilitate a favorable host/tissue response in terms of tissue overgrowth. An ideal host response to an implanted device will allow for rapid healing and endothelialization of device aspects proximate to tissue interfaces, while minimizing thrombus, fibrous overgrowth, pannus, and/or other inflammatory responses. However, as stated above, it is desirable to limit tissue overgrowth over pressure transmission elements (and, more specifically, over a diaphragm or other pressure-sensitive surface associated with the pressure transmission elements). Therefore, some embodiments of the devices disclosed herein can include a surface texturing (e.g., resulting from a sintered titanium manufacturing step) or a membrane covering (e.g., polyethylene terephthalate (PET) sold under the trademark DACRON, an expanded polytetrafluoroethylene (ePTFE) material, etc.) associated with one or more components. In some implementations, the surface treatment may be deployed in a specific, non-uniform manner in order to balance the desire for non-inflammatory tissue overgrowth in some sections of the device with the desire for limited tissue overgrowth over other aspects of the device. In further embodiments, the density, thickness, or another aspect of the surface treatment may vary across different sections of the device. Additionally, in some embodiments, a surface treatment can cover many sections of the device (e.g., cover clip sections, controller sections, most of a pressure transmission element section, etc.) while not covering other sections of the device (e.g., a pressure sensitive diaphragm or membrane at the distal portion of a pressure transmission element). In such embodiments, the surface treatment can terminate a short distance (e.g., 1 - 10 mm) away from a device aspect (e.g., a pressure sensitive diaphragm) that is desired to experience little to no tissue overgrowth in order to limit endothelialization and/or pannus formation on this portion of the device.

As one of skill in the art will appreciate from the disclosure herein, various components of the intracardiac pressure monitoring devices described above can be omitted without deviating from the scope of the present technology. Likewise, additional components not explicitly described above may be added to the intracardiac pressure monitoring devices without deviating from the scope of the present technology. Accordingly, the devices and systems described herein are not limited to those configurations expressly identified, but rather encompasses variations and alterations of the described devices and systems.

### Conclusion

Embodiments of the present disclosure may include some or all of the following components: a battery, supercapacitor, or other suitable power source; a microcontroller, FPGA, ASIC, or other programmable component or system capable of storing and executing software and/or firmware that drives operation of an implant; memory such as RAM or ROM to store data and/or software/firmware associated with an implant and/or its operation; wireless communication hardware such as an antenna system configured to transmit via Bluetooth, WiFi, or other protocols known in the art; energy harvesting means, for example a coil or antenna which is capable of receiving and/or reading an externally-provided signal which may be used to power the device, charge a battery, initiate a reading from a sensor, or for other purposes. Embodiments may also include one or more sensors, such as pressure sensors, impedance sensors, accelerometers, force/strain sensors, temperature sensors, flow sensors, optical sensors, cameras, microphones or other acoustic sensors, ultrasonic sensors, ECG or other cardiac rhythm sensors, SpO2 and other sensors adapted to measure tissue and/or blood gas levels, blood volume sensors, and other sensors known to those who are skilled in the art. Embodiments may include portions that are radiopaque and/or ultrasonically reflective to facilitate image-guided implantation or image guided procedures using techniques such as fluoroscopy, ultrasonography, or other imaging methods. Embodiments of the system may include specialized delivery catheters/systems that are adapted to deliver an implant and/or carry out a procedure. Systems may include components such as guidewires, sheaths, dilators, and multiple delivery catheters. Components may be exchanged via over-the-wire, rapid exchange, combination, or other approaches.

The above detailed description of embodiments of the technology are not intended to be exhaustive or to limit the technology to the precise forms disclosed above, but the scope of protection is rather defined by the claims. The various embodiments described herein may also be combined to provide further embodiments. For example, although this disclosure has been written to describe devices that are generally described as being used to create a path of fluid communication between the LA and RA, the LV and the right ventricle (RV), or the LA and the coronary sinus, it should be appreciated that similar embodiments could be utilized for shunts between other chambers of heart or for shunts in other regions of the body.

From the foregoing, it will be appreciated that specific embodiments of the technology have been described herein for purposes of illustration, but well-known structures and functions have not been shown or described in detail to avoid unnecessarily obscuring the description of the embodiments of the technology. Where the context permits, singular or plural terms may also include the plural or singular term, respectively.

## Claims

1. A device (200), comprising:
a first pressure sensor (206a) configured to reside within a first chamber of a heart (RA) of a patient;
a pressure transmission element (207a) configured to extend from the first chamber through a septal wall (S) to a second chamber of the heart (LA) of the patient; and
a second pressure sensor (206b) configured to reside within the first chamber of the heart,
wherein, during operation, the pressure transmission element (207a) is configured to transmit pressure from the second chamber to the first pressure sensor (206a) residing within the first chamber and the first pressure sensor (206a) is configured to measure the transmitted pressure from the second chamber, and
wherein, during operation, the second pressure sensor (206b) is configured to measure pressure within the first chamber of the heart of the patient.

2. The device (200) of claim 1 wherein the first chamber is the right atrium (RA) and the second chamber is the left atrium (LA).

3. The device (200) of claim 1 wherein, during operation, the first and second pressure sensors (206a, 206b) are configured to enable bi-atrial pressure sensing in the patient while leaving a majority of the fossa ovalis (FO) unobstructed.

4. The device (200) of claim 1 wherein the pressure transmission element (207a) is configured to traverse an outer edge portion of the fossa ovalis (FO) and leave sufficient access in the fossa ovalis for subsequent transseptal or septal procedures without affecting operation of the device.

5. The device (200) of claim 1 wherein the pressure transmission element (207a) comprises a fluid-filled tube.

6. The device (200) of claim 1 wherein the pressure transmission element (207a) is configured to transmit measured pressure to the first pressure sensor (206a) while leaving a majority of the fossa ovalis (FO) unobstructed.

7. The device (200) of claim 5 wherein the pressure transmission element (207a) comprises an elongated fluid-filled tube having a diameter less than or equal to 4 mm.

8. The device (200) of claim 1, further comprising:
a clip structure (202) including a first clip segment (204a) and a second clip segment (204b),
wherein, when the clip structure (202) is implanted in the heart of the patient, the first clip segment (204a) is configured to engage a first side of a septal wall and the second clip segment (204b) is configured to engage a second, opposite side of the septal wall; and
wherein the pressure sensor (206a, 206b) is carried by the clip structure (202).

9. The device (200) of claim 8 wherein the first clip segment (204a) is configured to engage a left atrial side of the septal wall and the second clip segment (204b) is configured to engage a right atrial side of the septal wall.

10. The device (200) of claim 9 wherein the first and second clip segments (204a, 204b) are biased towards each other so as to apply a force against the septal wall (S) when the clip structure (202) is implanted in the heart of the patient.

11. The device (200) of claim 8 wherein the clip structure (202) further comprises a hinge segment (204c) connecting the first and second clip segments (204a, 204b), and wherein the clip structure (200) has a generally U-shaped configuration.

12. The device (200) of claim 8 wherein the one pressure transmission element is carried by one of the first or second clip segments.

13. The device (200) of claim 12 wherein, when the clip structure (202) is implanted in the heart of the patient, the pressure transmission element (207a) extends away from the septal wall (S).

14. The device (200) of claim 12 wherein the pressure transmission element (207a) has a shape generally corresponding to a shape of the first clip segment (204a) of the clip structure (202).

## Patentansprüche

1. Vorrichtung (200), umfassend:
einen ersten Drucksensor (206a), der dazu konfiguriert ist, sich innerhalb einer ersten Kammer eines Herzens (RA) eines Patienten zu befinden;
ein Druckübertragungselement (207a), das dazu konfiguriert ist, sich von der ersten Kammer durch eine Scheidewand (S) zu einer zweiten Kammer des Herzens (LA) des Patienten zu erstrecken; und
einen zweiten Drucksensor (206b), der dazu konfiguriert ist, sich innerhalb der ersten Kammer des Herzens zu befinden,
wobei das Druckübertragungselement (207a) im Betrieb dazu konfiguriert ist, einen Druck von der zweiten Kammer an den ersten Drucksensor (206a) zu übertragen, der sich innerhalb der ersten Kammer befindet, und der erste Drucksensor (206a) dazu konfiguriert ist, den übertragenen Druck von der zweiten Kammer zu messen, und
wobei der zweite Drucksensor (206b) im Betrieb dazu konfiguriert ist, einen Druck innerhalb der ersten Kammer des Herzens des Patienten zu messen.

2. Vorrichtung (200) nach Anspruch 1, wobei die erste Kammer das rechte Atrium (RA) ist und die zweite Kammer das linke Atrium (LA) ist.

3. Vorrichtung (200) nach Anspruch 1, wobei der erste und der zweite Drucksensor 206a, 206b) im Betrieb dazu konfiguriert sind, eine biatriale Druckerfassung in dem Patienten zu ermöglichen, während ein Großteil der Fossa ovalis (FO) unblockiert belassen wird.

4. Vorrichtung (200) nach Anspruch 1, wobei das Druckübertragungselement (207a) dazu konfiguriert ist, einen äußeren Randteil der Fossa ovalis (FO) zu durchqueren und einen ausreichenden Zugang in der Fossa ovalis für anschließende transseptale oder septale Eingriffe zu belassen, ohne den Betrieb der Vorrichtung zu beeinträchtigen.

5. Vorrichtung (200) nach Anspruch 1, wobei das Druckübertragungselement (207a) einen mit Fluid gefüllten Schlauch umfasst.

6. Vorrichtung (200) nach Anspruch 1, wobei das Druckübertragungselement (207a) dazu konfiguriert ist, einen gemessenen Druck an den ersten Drucksensor (206a) zu übertragen, während ein Großteil der Fossa ovalis (FO) unblockiert belassen wird.

7. Vorrichtung (200) nach Anspruch 5, wobei das Druckübertragungselement (207a) einen länglichen, mit Fluid gefüllten Schlauch mit einem Durchmesser kleiner als oder gleich 4 mm umfasst.

8. Vorrichtung (200) nach Anspruch 1, ferner umfassend:
eine Klammerstruktur (202), einschließend ein erstes Klammersegment (204a) und ein zweites Klammersegment (204b),
wobei, wenn die Klammerstruktur (202) in das Herz des Patienten implantiert ist, das erste Klammersegment (204a) dazu konfiguriert ist, eine erste Seite einer Scheidewand in Eingriff zu nehmen, und das zweite Klammersegment (204b) dazu konfiguriert ist, eine zweite,
entgegengesetzte Seite der Scheidewand in Eingriff zu nehmen; und
wobei der Drucksensor (206a, 206b) von der Klammerstruktur (202) getragen wird.

9. Vorrichtung (200) nach Anspruch 8, wobei das erste Klammersegment (204a) dazu konfiguriert ist, eine Seite des linken Atriums der Scheidewand in Eingriff zu nehmen, und das zweite Klammersegment (204b) dazu konfiguriert ist, eine Seite des rechten Atriums der Scheidewand in Eingriff zu nehmen.

10. Vorrichtung (200) nach Anspruch 9, wobei das erste und das zweite Klammersegment (204a, 204b) zueinander vorgespannt sind, um eine Kraft gegen die Scheidewand (S) auszuüben, wenn die Klammerstruktur (202) in das Herz des Patienten implantiert ist.

11. Vorrichtung (200) nach Anspruch 8, wobei die Klammerstruktur (202) ferner ein Gelenksegment (204c) umfasst, das das erste und das zweite Klammersegment (204a, 204b) verbindet, und wobei die Klammerstruktur (200) eine allgemein U-förmige Konfiguration aufweist.

12. Vorrichtung (200) nach Anspruch 8, wobei das eine Druckübertragungselement von einem von dem ersten und dem zweiten Klammersegment getragen wird.

13. Vorrichtung (200) nach Anspruch 12, wobei, wenn die Klammerstruktur (202) in das Herz des Patienten implantiert ist, sich das Druckübertragungselement (207a) von der Scheidewand (S) weg erstreckt.

14. Vorrichtung (200) nach Anspruch 12, wobei das Druckübertragungselement (207a) eine Form aufweist, die allgemein einer Form des ersten Klammersegments (204a) der Klammerstruktur (202) entspricht.

## Revendications

1. Dispositif (200), comprenant :
un premier capteur de pression (206a) configuré pour résider dans une première cavité cardiaque (RA) d'un patient ;
un élément de transmission de pression (207a) configuré pour s'étendre depuis la première cavité à travers une paroi septale (S) jusqu'à une seconde cavité cardiaque (LA) du patient ; et
un second capteur de pression (206b) configuré pour résider dans la première cavité cardiaque,
pendant le fonctionnement, l'élément de transmission de pression (207a) étant configuré pour transmettre la pression de la seconde cavité au premier capteur de pression (206a) situé dans la première cavité, et le premier capteur de pression (206a) étant configuré pour mesurer la pression transmise depuis la seconde cavité, et
pendant le fonctionnement, le second capteur de pression (206b) étant configuré pour mesurer la pression dans la première cavité cardiaque du patient.

2. Dispositif (200) selon la revendication 1, la première cavité étant l'oreillette droite (RA) et la seconde cavité étant l'oreillette gauche (LA).

3. Dispositif (200) selon la revendication 1, pendant le fonctionnement, les premier et second capteurs de pression (206a, 206b) étant configurés pour permettre la détection de la pression dans les deux oreillettes du patient tout en laissant une majeure partie de la fosse ovale (FO) dégagée.

4. Dispositif (200) selon la revendication 1, l'élément de transmission de pression (207a) étant configuré pour traverser une partie formant bord extérieur de la fosse ovale (FO) et laisser un accès suffisant dans la fosse ovale pour des procédures transseptales ou septales ultérieures sans affecter le fonctionnement du dispositif.

5. Dispositif (200) selon la revendication 1, l'élément de transmission de pression (207a) comprenant un tube rempli de fluide.

6. Dispositif (200) selon la revendication 1, l'élément de transmission de pression (207a) étant configuré pour transmettre la pression mesurée au premier capteur de pression (206a) tout en laissant une majeure partie de la fosse ovale (FO) dégagée.

7. Dispositif (200) selon la revendication 5, l'élément de transmission de pression (207a) comprenant un tube rempli de fluide allongé présentant un diamètre inférieur ou égal à 4 mm.

8. Dispositif (200) selon la revendication 1, comprenant en outre :
une structure de pince (202) comprenant un premier segment de pince (204a) et un second segment de pince (204b),
lorsque la structure de pince (202) est implantée dans le cœur du patient, le premier segment de pince (204a) étant configuré pour s'engager sur un premier côté d'une paroi septale et le second segment de pince (204b) étant configuré pour s'engager sur un second côté opposé de la paroi septale ; et
le capteur de pression (206a, 206b) étant porté par la structure de pince (202).

9. Dispositif (200) selon la revendication 8, le premier segment de pince (204a) étant configuré pour s'engager sur un côté auriculaire gauche de la paroi septale et le second segment de pince (204b) étant configuré pour s'engager sur un côté auriculaire droit de la paroi septale.

10. Dispositif (200) selon la revendication 9, les premier et second segments de pince (204a, 204b) étant sollicités l'un vers l'autre de manière à exercer une force contre la paroi septale (S) lorsque la structure de pince (202) est implantée dans le cœur du patient.

11. Dispositif (200) selon la revendication 8, la structure de pince (202) comprenant en outre un segment de charnière (204c) reliant les premier et second segments de pince (204a, 204b), et la structure de pince (200) présentant une configuration généralement en forme de U.

12. Dispositif (200) selon la revendication 8, l'élément de transmission de pression étant porté par l'un des premier et second segments de pince.

13. Dispositif (200) selon la revendication 12, lorsque la structure de pince (202) est implantée dans le cœur du patient, l'élément de transmission de pression (207a) s'étendant à l'écart de la paroi septale (S).

14. Dispositif (200) selon la revendication 12, l'élément de transmission de pression (207a) présentant une forme correspondant généralement à une forme du premier segment de pince (204a) de la structure de pince (202).
